# EUROPÄISCHE PATENTSCHRIFT

(11) **EP 1 868 141 B1**
(45) Veröffentlichungstag und Bekanntmachung des Hinweises auf die Patenterteilung: **09.11.2016**
(21) Anmeldenummer: 07116846.2
(22) Anmeldetag: 23.08.2005
(51) Int. Cl.: G06M 1/16, G06M 1/22, G06M 1/04, A61M 15/00, B65D 83/14, G06M 1/08, G06M 1/24

(54) **Inhalier-Gerät**
Inhaler
Inhalateur

(30) Priorität: 10.11.2004 DE 102004054179; 18.07.2005 DE 102005033398
(43) Veröffentlichungstag der Anmeldung: 19.12.2007
(62) Teilanmeldung aus: 05792078.7
(73) Patentinhaber: Von Schuckmann, Alfred, 47627 Kevelaer (DE)
(72) Erfinder: Von Schuckmann, Alfred, 47627 Kevelaer (DE)
(74) Vertreter: Rieder, Hans-Joachim

(56) Entgegenhaltungen:
- EP-A- 1 065 477
- US-A- 3 107 855
- US-A1- 2003 178 020
- US-A1- 2004 144 798
- US-A1- 2004 149 772
- US-A1- 2004 211 420
- US-B1- 6 431 168

## Beschreibung

Die Erfindung betrifft ein Handgerät nach dem Merkmal des Oberbegriffes des Anspruches 1.

Handgeräte der in Rede stehenden Art sind vorbekannt (US-PS 2003/0178020). Sie finden insbesondere Anwendung in der medizinischen Aerosol-Therapie zur Behandlung von Erkrankungen der Atemwege. Die im Gehäuse gehalterte, unter Druck stehende Kartusche beinhaltet das zu inhalierende Medikament, wobei zur Freigabe bzw. zum Ausstoß desselben eine axiale Verschiebung der Kartusche in dem Gehäuse erforderlich ist. Da bei jeder Kartuschenbetätigung nur eine definierte Medikamentenmenge abgegeben wird, ist es bekannt, die Zählvorrichtung dadurch für den Benutzer übersichtlich zu machen, dass mehrere Skalenringe vorgesehen sind, von denen der erste Ring Stück für Stück bis zehn zählt und einen zweiten Ring betätigt, auf dem die Anzahl der Zehnerschritte angezeigt ist, und dieser eventuell sogar noch nach zehnmaliger Schrittbetätigung einen weiteren Skalenring anspricht, der die Hunderter festhält usw. Die Lösung bringt zwar übersichtliche Skaleneinteilungen auf dem Skalenring. Sie ist aber äußerst aufwendig und bedienungsunfreundlich.

Zu einem derartigen Handgerät ist als Stand der Technik beispielsweise auf die US2004/149772A1 zu verweisen. Aus der US 3,107,855 ist aus einem Planetenrad und einem Sonnenrad bei einer Zelleinrichtung zusammengesetztes Getriebe bekannt.

Ausgehend von dem genannten Stand der Technik beschäftigt sich die Erfindung mit der Aufgabenstellung, eine günstige Ausgestaltung eines derartigen Handgerätes hinsichtlich des Zählwerks anzugeben.

Diese Aufgabe ist beim Gegenstand des Anspruches 1 gelöst, wobei darauf abgestellt ist, dass ein Planetenrad einen einseitig von einer Scheibe eines Sonnenrades mit abragenden Achszapfen aufweist, dass der Achszapfen in einer Bohrung in Bereich eines scheibenartig nach radial innen weisenden Kragens eines Ringes drehbar gefasst ist, dass der Mantel ausseitig mit einer umlaufenden Skaleneinteilung versehen ist, wobei die Skaleneinteilung jeweils mehreren Einzel-Drehschritten des den Skalenring weiterführenden Planetenrades entspricht und dass die Drehung des Planetenrades um dessen Achse und damit einhergehende Drehverlagerung des Skalenringes erst nach mehreren Einzel-Drehschritten des Sonnenrades durchgeführt wird.

Das so ausgebildete Handgerät ist durch die gewählte Konstruktion sowohl hinsichtlich des Platzbedarfs für das Schaltwerk als auch hinsichtlich der Reirigungsmöglichkeiten und darüber hinaus auch hinsichtlich der Handhabbarkeit in vorteilhafter Weise ausgebildet ist. Es ist ein mechanisch arbeitendes Schrittschaltwerk vorgesehen, welches aufgrund der gewählten Anordnung eine günstige, raumsparende Bauform aufweist. Man kommt mit einem Skalenring aus und selbst der braucht nicht mit kaum zu erkennbaren Strichunterteilungen versehen zu sein. Man kann z.B. zwischen zwei Skalenstrichen oder Zeichen immer eine bestimmte Anzahl von Entnahmebetätigungen vornehmen, ohne dass der Skalenring sich dreht. Man kann diese Handhabung auch in Abhängigkeit von bestimmten Medikamenten festlegen, z.B. wenn ein Medikament jeweils in drei Betätigungen eingegeben werden muss/soll. Die erste Eingabe ist auf der Skala dann erkennbar. Die zweite und dritte aber nicht als Drehung des Skalenringes verkörpert. Die Funktion ist nur durch rein mechanisch zusammenwirkende Bauteile gewährleistet. Dies trägt weiter zu einer vereinfachten Herstellbarkeit und kleiner Bauform bei. Die Lösung, dass das Planetenrad in einer Bohrung des Skalenrings lagert und das zugehörige Sonnenrad auf einer unterseitig gezahnten Scheibe sitzt, minimiert die Bauteile. Diese Scheibe steht in Eingriff mit dem Schaltfinger. Dieser greift in die Zahnung ein und ein Rastfinger sichert die jeweils erreichte ScheibenDrehstellung. Sonnenrad und gezahnte Scheibe sind einteilig ausgeformt. Entsprechend sind auf dem Skalenring angepasst an das Kartuschenvolumen bspw. 200 oder 300 Hubstöße deutlich anzeigbar. Die auf der äußeren Mantelfläche des Skalenrings angeordnete und vor einem Sichtfenster des Gehäuses laufende Skaleneinteilung des Skalenrings entspricht jeweils mehreren Einzel-Drehschritten des Planetenrades. Diesbezüglich erweist es sich als vorteilhaft, dass hinsichtlich der Untersetzung ein Einzel-Drehschritt des Planetenrades erst nach Durchlauf mehrerer Einzel-Drehschritte des Sonnenrades erfolgt.

Nachstehend ist der Gegenstand der Erfindung anhand der beigefügten Zeichnung, welche ein Ausführungsbeispiel darstellt, näher erläutert. Es zeigen:
- Fig. 1: in perspektivischer Explosionsdarstellung das Schrittschaltwerk für das erfindungsgemäße Handgerät;

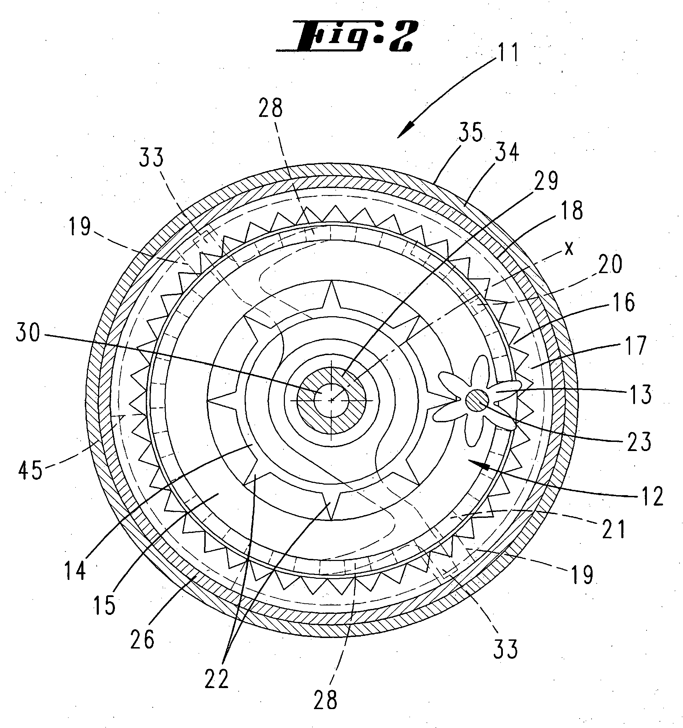

- Fig. 2: einen Querschnitt durch das Schrittschaltwerk;
- Fig. 3: in einer Längsschnittdarstellung das Handgerät in einer Ausführungsform, mit einem von einer Kartusche verrasteten, schematisch dargestellten Schrittschaltwerk,
- Fig. 4: eine Bauform, bei welcher das Schrittschaltwerk nicht mit der Kartusche verrastet ist.

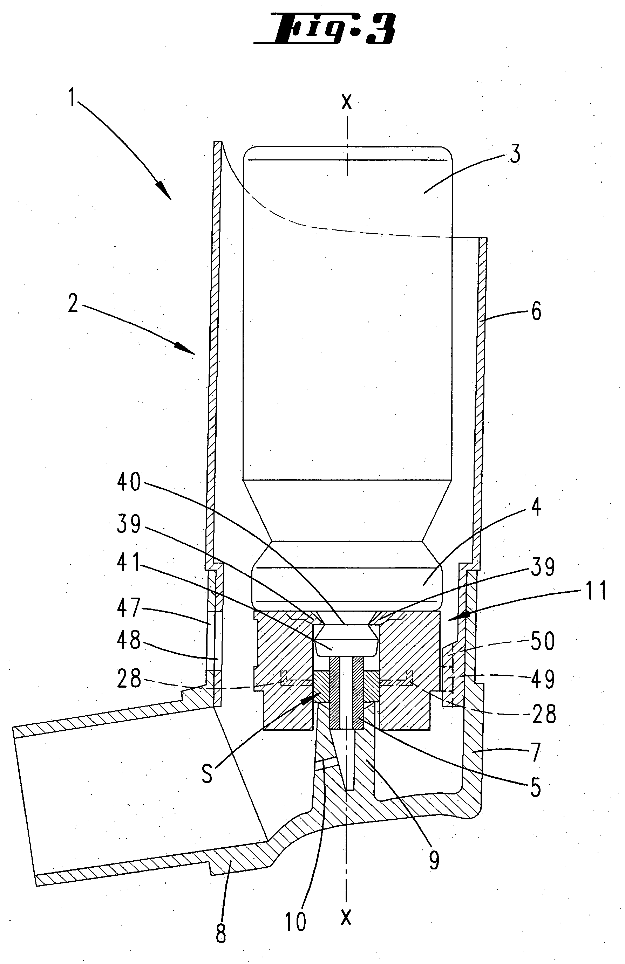

Das in Fig. 3 in einer schematischen Schnittdarstellung gezeigte Handgerät 1 dient zur portionierten Ausgabe sprühfähiger Substanzen, insbesondere von Inhaliermedikamenten.

Hierzu weist das Handgerät 1 zunächst ein Handgerätgehäuse 2 auf, in welches eine die sprühfähige Substanz beinhaltende Kartusche 3 einsetzbar ist. Diese Kartusche 3 ist in dem Gehäuse 2 axial verschiebbar.

In üblicher Weise weist der Kartuschenkopf 4 ein zentrales, sich koaxial zur Kartusche 3 erstreckendes Ventilrohr 5 auf. Über letzteres wird eine Medikamentenausgabe durch eine axiale Relativbewegung zwischen Kartusche 3 und Gehäuse 2 erreicht.

Das Gehäuse 2 ist zweigeteilt und besteht im Wesentlichen aus zwei übereinander angeordneten Ringteilen 6 und 7, von welchen das obere Ringteil 6 schaftartig ausgeformt ist und das untere Ringteil 7 ein etwa quer zur Schafterstreckung ausgerichtetes Mundstück 8 aufweist. Letzteres ist durch eine nicht dargestellte Abdeckkappe verschließbar.

Das Ventilrohr 5 der Kartusche 3 stützt sich in einem zugeordneten rohrförmigen Stützabschnitt 9 innerhalb des unteren Ringteiles 7 ab, dies bei axialer Beweglichkeit der Kartusche 3 innerhalb des die Kartusche 3 umgebenden, schaftartigen Ringteils 6.

Der das Ventilrohr 5 der Kartusche 3 klemmend aufnehmende, innerhalb des unteren Gehäuse-Ringteils 7 ausgeformte Stützabschnitt 9 ist mit einem gegenüber einen das Ventilrohrende aufnehmenden Abschnitt durchmesserverringerten Strömungskanal 10 versehen, welcher strömungstechnisch in Verbindung steht mit dem Ventilrohr 5, wobei das dem Ventilrohr 5 abgewandte Ende des Strömungskanals 10 in Richtung auf das Mundstück 8 weist.

Die beiden Ringteile 6 und 7 sind in dem dargestellten Ausführungsbeispiel steckbar miteinander verbunden. Alternativ können die beiden Teile auch über ein Gewinde, bspw. über ein Grobgewinde mit hoher Steigung, miteinander verbunden sein.

Die Anordnung der Kartusche 3 in dem Gehäuse 2 ist so gewählt, dass der Kartuschenkopf 4 in dem Gehäuse 2 etwa auf Höhe des Verbindungsbereiches zwischen Ringteil 6 und Ringteil 7 platziert ist.

Zentral unterhalb der öffnungsseitigen Stirnwand der Kartusche 3 ist in Überlappung zum Kartuschen-Ventilrohr 5 ein Schrittschaltwerk 11 angeordnet. Dieses dient zum Registrieren und Anzeigen der durchgeführten Ausgabebetätigungen, dies in Abhängigkeit von den durchgeführten Öffnungshüben der Kartusche 3.

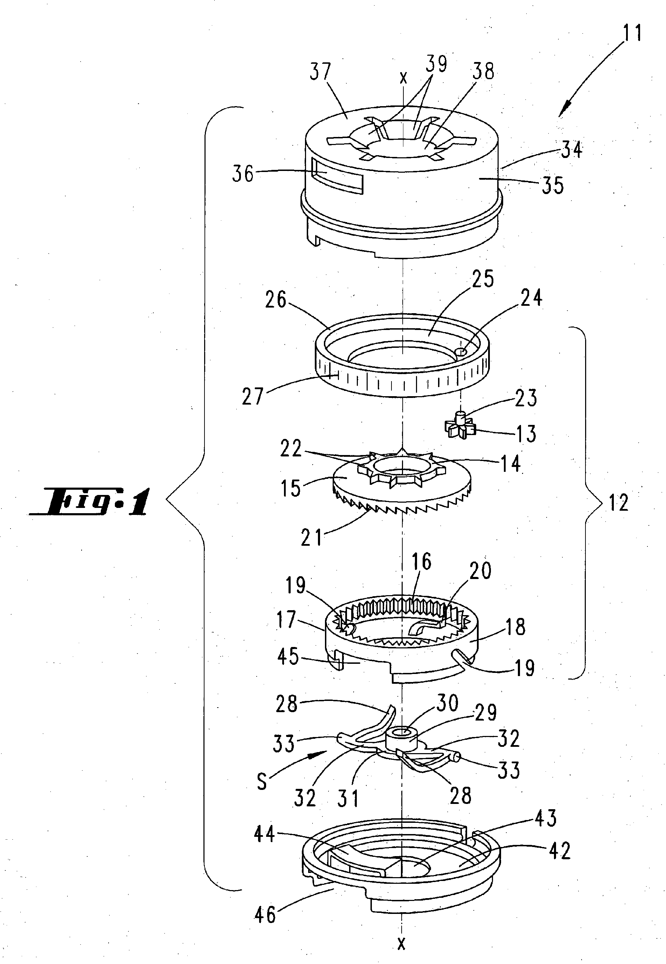

Das Schrittschaltwerk 11 ist in Fig. 1 in einer perspektivischen Explosionsdarstellung gezeigt. Zentraler Bestandteil des Schrittschaltwerks 11 ist ein Planetenrad-Getriebe 12, bestehend aus einem Planetenrad 13, einem Sonnenrad 14, welches auf einer unterseitig gezahnten Scheibe 15 sitzt und einem mit dem Planetenrad 13 zusammenwirkenden Zahnkranz 16. Letzterer ist wandungsinnenseitig eines nicht drehbar gehalterten, rohrabschnittförmigen Ringes 17 ausgeformt. Die Mantelwandung 18 des Ringes 17 ist in diametral gegenüberliegenden Bereichen durchsetzt von schräg aufwärts in Schaltrichtung gerichtet ausgehenden Schlitzen 19, die nach unten zur dem Zahnkranz 16 abgewandten Ringkante hin offen auslaufen.

Der Zahnkranz 16 erstreckt sich in axialer Richtung etwa über die halbe Höhe des Ringes 17, dessen Mantelwandung 18 zur dem Zahnkranz 16 abgewandten Ringstirnkante hin abgestuft, sich radial verjüngend ausgebildet ist.

Unterhalb des Zahnkranzes 16 ist innenseitig der Mantelwandung 18 des Ringes 17 ein Rastfinger 20 angeformt. Dieser ist mit Bezug auf einen Grundriss des Ringes gegenüber dem Zahnkranz 16 nach radial innen versetzt; greift entsprechend in einen zum Zahnkranz 16 nach radial innen eingezogenen Kreisraum. Weiter ist die Anordnung des in etwa in Vertikalrichtung elastisch ausgebildeten Rastfingers 20 so gewählt, dass dieser etwa in eine durch die unteren Randkanten des Zahnkranzes 16 aufgespannte Horizontalebene eingreift.

Der Durchmesser der das Sonnenrad 14 tragenden Scheibe 15 ist geringfügig kleiner gewählt, als der Innendurchmesser des Ringes 17 im Bereich des Zahnkranzes 16. Sonnenrad 14 und Scheibe 15 sind bevorzugt einstückig, materialeinheitlich ausgebildet.

Unterseitig der Scheibe 15 ist eine randbezogen umlaufende Sägezahnung 21 vorgesehen, in welche der Rastfinger 20 des Ringes 17 eingreift.

Das Sonnenrad 14 weist eine grobe Verzahnung auf. So sind in dem dargestellten Ausführungsbeispiel über den Umfang des Sonnenrades 14 acht Sonnenrad-Zähne 22 gleichmäßig verteilt ausgeformt. Diese Zähne 22 wirken im Zuge der Sonnenrad-Umdrehung mit dem in derselben Ebene zwischen dem Sonnenrad 14 und dem Zahnkranz 16 des Ringes 17 angeordneten Planetenrad 13 zusammen.

Das Planetenrad 13 besitzt einen einseitig nach oben, d. h. von der Scheibe 15 des Sonnenrades 14 weg abragenden Achszapfen 23. Dieser ist in einer Bohrung 24 im Bereich eines scheibenartig nach radial innen weisenden Kragens 25 eines Skalenringes 26 drehbar gefasst. Der Skalenring 26 ist mantelaußenwandig mit einer umlaufenden Skaleneinteilung 27 versehen, wobei die Skaleneinteilung jeweils mehreren Einzel-Drehschritten des den Skalenring 26 weiterführenden Planetenrades 13 entspricht.

Die schrittweise Verlagerung des Sonnenrades 14 bzw. der einstückig hiermit ausgeformten Scheibe 15 erfolgt über etwa vertikal federnd ausweichbar ausgebildete Schrittschaltfinger 28. Diese greifen unterseitig in die Sägezahnung 21 der Scheibe 15.

Die Schrittschaltfinger 28 sind mit Bezug zu der Hauptachse x des gesamten Schrittschaltwerkes 11 diametral gegenüberliegend angeordnet. Hierzu ist zunächst ein zylinderförmiger Zentralkörper in Form einer Nabe 29 vorgesehen mit einer zentralen axialen Durchgangsbohrung 30. Deren Durchmesser ist geringfügig größer bemessen als der Außendurchmesser des diese Durchgangsbohrung 30 zu durchsetzenden Kartuschen-Ventilrohres 5.

Fußseitig geht die Nabe 29 über in einen radial erweiterten Kragen 31. An diesem sind diametral gegenüberliegend in Radialrichtung abragende Führungsabschnitte 32 angeformt, die jeweils im Bereich ihrer freien Enden einen in dem zugeordneten Schlitz 19 des Ringes 17 einliegenden Führungszapfen 33 ausformen.

Die Schrittschaltfinger 28 wurzeln jeweils mit einem Horizontalabschnitt an den Führungsabschnitten 32 unter Belassen der über den horizontalen Abschnitt radial außen abragenden Führungszapfen 33. Die von den Horizontalabschnitten abragenden Schrittschaltfinger 28 gehen schräg aufwärts gerichtet aus, etwa unter Einschließen eines Winkels von 45 Grad zur Horizontalen, angepasst an die Schrägung der Schlitze 19 im Ring 17. Der so gebildete Schrittschaltfinger-Stern trägt das Bezugszeichen S.

Der Schrittschaltfinger-Stern S, der den inneren Zahnkranz 16 aufweisende Ring 17, die einstückig mit dem Sonnenrad 14 ausgeformte Scheibe 15 sowie der Skalenring 26 sind konzentrisch zueinander auf der Achse x ausgerichtet, wobei die Höhe des Ringes 17 so gewählt ist, dass sowohl der Schrittschaltfinger-Stern S als auch das Sonnenrad 14 samt Scheibe 15 in diesem aufgenommen sind.

Das gesamte Planeten-Getriebe 12, sowie der Schrittschaltfinger-Stern S und der Skalenring 26 sind aufgenommen in einem topfartigen Schrittschaltwerkgehäuse 34 mit einem Außendurchmesser, welcher an den Außendurchmesser der Kartusche 3 angepasst ist.

Das Gehäuse 34 besitzt eine Mantelwandung 35. Diese weist ein Sichtfenster 36 auf, durch welches die Skaleneinteilung 27 des Skalenrings 26 erkennbar ist.

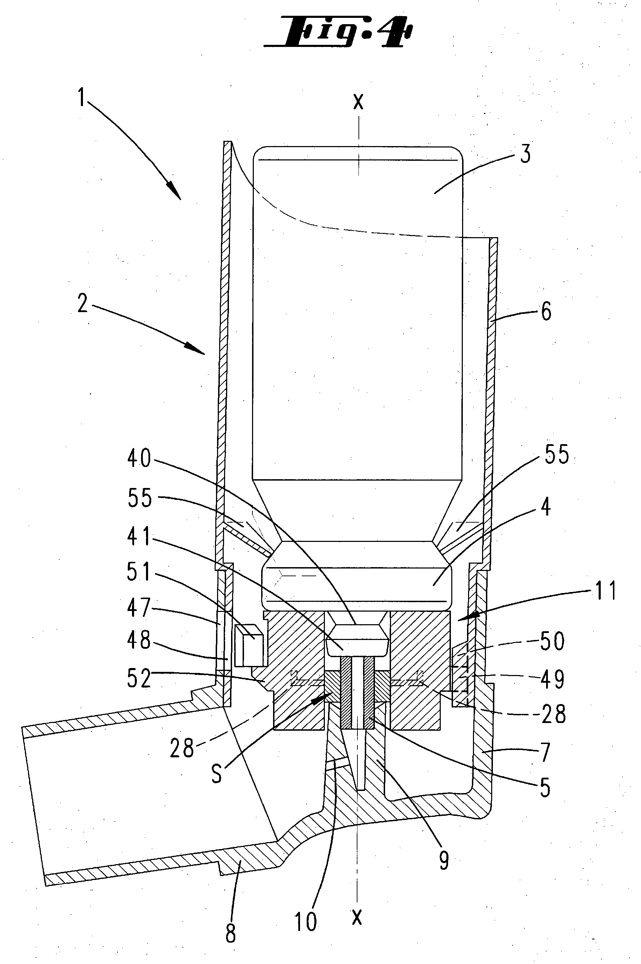

Die Gehäusedecke 37 besitzt eine zentrale Durchbrechung 38, welche in der in den Figur 1 gezeigten Ausführungsform umfasst ist von konisch zum Gehäuseinnern hin zulaufenden Rast-Federzungen in der in de Figur 1 gezeigten Ausführungsform 39. Der Durchbrechungs-Durchmesser ist angepasst an einen Durchmesser eines Taillenabschnittes 40 eines über die öffnungsseitige Stirnwand der Kartusche 3 zentral überragenden Kragens 41, aus welch letzterem das Ventilrohr 5 auswächst. Wie in Figur 4 dargestellt, kann das Gehäuse aber auch unabhängig von der Kartusche 3 eingesetzt sein.

Die Darstellung in Figur 4 zeigt eine weitere Ausführungsform. Dort ist zur weiteren Festlegung der Kartusche 3 - neben einer üblichen Klemmhalterung des Ventilrohrs 5 in dem handgerätgehäuseseitigen Stützabschnitt 9 - eine Blockierung der Kartusche 3 im Bereich des oberen Ringteil-Gehäuses 6 vorgesehen. So ragen mantelinnenseitig dieses oberen Gehäuseringteiles 6 schräg abwärts gerichtete Rückhaltefinger 55 ab, welche materialeinheitlich, einstückig mit dem oberen Gehäuseteil 6 gebildet sind. Diese Rückhaltefinger 55 sind so positioniert, dass deren freien Randkanten in der Zuordnungsstellung zur Kartusche 3 in den hinter dem Kartuschenkopf 4 ausgeformten Taillenbereich der Kartusche 3 sperrend eintreten, um so die Kartusche 3 zu blockieren. Weiter sind die Rückhaltefinger 55 derart ausgeformt, dass diese zumindest bei einer Erstbestückung des Gehäuses 2 mit der Kartusche 3 durch den Kartuschenkopf 4 überlaufen werden können. Gezählt wird bei diesem Einsetzen nicht. Die Rückstellung der betätigten Kartusche übernimmt deren Ventilrohr-Feder und diejenige des Schrittschaltwerkes die Federkraft der Schrittschaltfinger 28.

Der Gehäuseboden 42 ist gebildet durch ein gesondertes Teil. Dieses ist unter Aufnahme der vorbeschriebenen Schrittschaltwerk-Einzelteile mit dem Gehäuse 34 verbunden, so bspw. mit diesem verschweißt oder über eine Presspassung an diesem gehaltert.

Der tellerartige Gehäuseboden 42 besitzt eine zentrale Bohrung zum Durchtritt des Ventilrohres 5. Des Weiteren ist auf dem Gehäuseboden 42 ein Raststück 44 ausgeformt, welches zur lageorientierten Fesselung des Ringes 17 in eine entsprechend in dessen Mantelwandung 18 ausgeformte, fensterartige Ausnehmung 45 greift.

Im selben Winkelbereich, in welchem das Passstück 44 auf dem Boden angeordnet ist, weist die Mantelaußenwandung des Gehäusebodens 42 einen Freischnitt 46 auf. Diese ist im Einbauzustand dem Bereich des Ausgangsquerschnittes des Strömungskanals 10 im unteren Ringteilgehäuse 7 zugeordnet.

Die Funktionsweise des Schrittschaltwerkes 11 ist wie folgt:

Die Schaltglieder (Schriftschaltfinger-Stern S, Ring 17, Scheibe 15, Planetenrad 13 und Skalenring 26) wie auch das Gehäuse 34 mit dem Gehäuseboden 42 sind auf Achsen angeordnet, die sich in Längsrichtung der Kartusche 3 erstrecken. So sind mit Ausnahme des Planetenrades 13 alle weiteren Bauteile des Schrittschaltwerkes 11 auf der Kartuschenlängsachse x - x positioniert.

Das Schrittschaltwerk 11 ist entsprechend konzentrisch zum Ventilrohr 5 im Schatten der Kartusche 3 innerhalb des Gehäuses 2 angeordnet, dies konkret in dem zwischen Kartuschenkopf 4 und Stützabschnitt 9 des Gehäuses 2 belassenen Bauraum. Das Schrittschaltwerk 11 stützt sich mit der in dem Schaltwerkgehäuse 34 zentral gelagerten Nabe 29 des Schrittschaltfinger-Sterns S auf der Stirnfläche des Stützabschnitts 9 des Inhaliergehäuses 2 ab. Die zentrale Achse x des Schnittschaltwerks 11 wird von der Körperachse der Kartusche 3 übernommen. Das die Nabe 29 durchsetzende Ventilrohr 5 bietet eine zusätzliche Zentrierung der gesamten Schrittschaltwerk-Einheit.

Bei Durchführung eines Betätigungshubs der Kartusche 3 und damit einhergehender Vertikalverlagerung derselben in Richtung auf den Stützabschnitt 9 wird das Schaltwerkgehäuse 34 über den Kartuschenkopf 4 mitgeschleppt, dies unter Relativverlagerung des Gehäuses 34, des Planetenrad-Getriebes 12 und des Skalenringes 26 zu dem Schrittschaltfinger-Stern S, welcher eine Abstützung auf dem Stützabschnitt 9 erfährt. Infolgedessen bewirken die sich spannenden Schrittschaltfinger 28 durch Drehaufgleiten des Schrittschaltfinger-Sterns S in den mantelwandseitigen Schlitzen 19 des Ringes 17 einen schrittweisen Drehvorschub der sägeverzahnten Scheibe 15. Einhergehend damit dreht sich das Sonnenrad 14 um denselben Winkelbetrag. Die Schrittschaltfinger 28 bewegen sich hierbei aus der schrägen Ausrichtung in Richtung auf eine senkrecht zur Längsachse x - x ausgerichtete Ebene.

Dadurch bedingt, dass das Sonnenrad 14 lediglich acht gleichmäßig über den Umfang verteilt angeordnete Zähne aufweist, führt nicht jede Schritt-Drehbewegung des Sonnenrades 14 zwangsläufig zu einer Drehbewegung des Planetenrades 13. Vielmehr wird die Drehung des Planetenrades 13 um dessen Achse und eine die damit einhergehende Drehverlagerung des Skalenringes 26 erst nach mehreren Einzel-Drehschritten des Sonnenrades 14 durchgeführt.

Gemäß der Darstellung in Fig. 3 ist das gesamte Schrittschaltwerk 11 mittels des Gehäuses 34 an dem kartuschenkopfseitig vorragenden Kragen 41 rastend festlegbar. Zugeordnet zu dem gehäuseseitigen Sichtfenster 36 weisen die zugeordneten Abschnitte der Gehäuseringteile 6 und 7 gleichfalls Sichtfenster 47, 48 auf, welche durch die gewählte Position, zugewandt dem Mundstück 8 des Gehäuses 2, im Blickfeld des das Handgerät 1 bedienenden Benutzers liegen. Zur lageorientierten Einführung des Schrittschaltwerkes 11 ist dieses mit einem radial von dem Gehäuse 34 abragenden Führungsblatt 49 versehen, welches in eine nicht näher dargestellte Vertikalnut im Innern des Gehäuses 2, den Verschiebeweg bei Hubbetätigung zulassend eingreift.

Die Verrastung zwischen Schrittschaltwerk 11 und Kartusche 3 im Bereich des kartuschenkopfseitigen Kragens 41 ist so gewählt, dass bei einer Entnahme der Kartusche 3 aus dem Gehäuse 2 das Schrittschaltwerk 11 an der Kartusche 3 verbleibend mit ausgezogen wird.

## Patentansprüche

1. Handgerät (1) zur portionierten Ausgabe sprühfähiger Substanzen, insbesondere Inhaliermedikamenten, mit einer durch Drücken in einem Gehäuse (2) in die Ausgabe-Öffnungs-Stellung verschiebbaren Kartusche (3) und einem beim Öffnungshub der Kartusche (3) von dieser mitbewegten Schaltwerk (11) zum Registrieren und Anzeigen durchgeführter Ausgabebetätigungen, welches Schaltwerk (11) in einem Gehäuse (34) zentral unterhalb der öffnungsseitigen Stirnwand der Kartusche (3) überlappend zu einem Kartuschen-Ventilrohr (5) angeordnet ist, welches Schaltwerk ein in einem tellerförmigen Gehäuse (34) angeordnetes Schrittschaltwerk (11) mit Schaltfingern (S) ist, welche Schaltfinger um in Längsrichtung der Kartusche (3) liegende Achsen umlaufen, **dadurch gekennzeichnet, dass** ein Planetenrad (13) einen einseitig von einer Scheibe (15) eines Sonnenrades (14) weg abragenden Achszapfen (23) aufweist, dass der Achszapfen (23) in einer Bohrung (24) im Bereich eines scheibenartig nach radial innen weisenden Kragens eines Ringes (26) drehbar gefasst ist, dass der Ring (26) mantelaußenseitig mit einer umlaufenden Skaleneinteilung (27) versehen ist, wobei die Skaleneinteilung jeweils mehreren Einzel-Drehschritten des den Skalenring (26) weiterführenden Planetenrades (13) entspricht und dass die Drehung des Planetenrades (13) um dessen Achse und die damit einhergehende Drehverlagerung des Skalenringes (26) erst nach mehreren Einzel-Drehschritten des Sonnenrades (14) durchgeführt wird.

## Claims

1. Hand-held device (1) for apportioned delivery of sprayable substances, in particular inhaler medicaments, having a cartridge (3) which is displaceable by pressing in a housing (2) into the open position for delivery, and an indexing mechanism (11) for registering and displaying delivery actuations which have been carried out, which indexing mechanism is moved by and along with the cartridge (3) during the opening stroke of the cartridge, which indexing mechanism (11) is disposed in a housing (34) centrally below the opening-side end wall of the cartridge (3), overlappingly with respect to a valve tube (5) of the cartridge, which indexing mechanism is a stepby-step indexing mechanism (11) having indexing-fingers (S) and is located in a cup-like housing (34), which indexing-fingers encircle axes which lie in the longitudinal direction of the cartridge (3), **characterized in that** a planet wheel (13) has a spindle (23) which protrudes on one side away from the disk (15) of a sun wheel (14), that the spindle (23) is held rotatably in a hole (24) in the region of a collar, which is directed radially inwards in the manner of a disk, of a ring (26), that the ring (26) is provided on its outer peripheral wall with a circumferential graduation (27), the graduation corresponding in each case to a plurality of individual rotary steps of the planet wheel (13) driving the graduated ring (26) onwards, and that the rotation of the planet wheel (13) about its axis and the rotational displacement of the graduated ring (26) associated therewith are carried out only after a plurality of individual rotational steps of the sun wheel (14).

## Revendications

1. Dispositif manuel (1) de délivrance fractionnée de substances pulvérisables, en particulier des médicaments à inhaler, comprenant une cartouche (3) qui, par appui, est déplaçable dans un boîtier (2) vers une position d'ouverture de délivrance et un mécanisme d'indexage (11) se déplaçant avec la cartouche lors de la course d'ouverture de la cartouche pour indexer et indiquer les actionnements de délivrance réalisés, lequel mécanisme d'indexation (11) est agencé dans un boîtier (34) centralement sous la paroi avant de la cartouche (3) du côté de son ouverture, en chevauchement d'un tube de valve (5) de la cartouche, lequel mécanisme d'indexation est un mécanisme d'indexation pas-à-pas (11) ayant des doigts d'indexation (S) et qui agencé dans un boîtier (34) en forme d'assiette, lesquels doigts d'indexation tournent autour d'axes s'étendant en direction longitudinale de la cartouche (3), **caractérisé en ce qu'**une roue satellite (13) présente un tourillon (23) qui fait saillie du côté opposé à un disque (15) d'un roue planétaire intérieure (14), que le tourillon (23) est logé à rotation dans un alésage (24) dans la région d'un col d'une bague (26) lequel s'étend radialement vers l'intérieur à la façon d'un disque, que la bague (26) est pourvue de graduations circonférentielles sur sa surface extérieure, dans lequel les graduations correspondent chacune à plusieurs pas de rotation individuels de la roue satellite (13) qui entraine la bague graduée (26) et que la rotation de la roue satellite (13) autour de son axe, ainsi que le déplacement en rotation de la bague graduée (26) qui en résulte ne se produit qu'après plusieurs pas de rotation individuels de la roue planétaire intérieure (14).
